# EUROPÄISCHE PATENTSCHRIFT

(11) **EP 1 935 374 B1**
(45) Veröffentlichungstag und Bekanntmachung des Hinweises auf die Patenterteilung: **29.06.2011**
(21) Anmeldenummer: 07024709.3
(22) Anmeldetag: 20.12.2007
(51) Int. Cl.: A61F 2/06

(54) **Schlauchförmige Gefäßprothese zum Ersatz der Aorta ascendens**
Tube-like stent to replace the aorta ascendens
Prothèse vasculaire tubulaire destinée à remplacer l'aorte ascendante

(30) Priorität: 22.12.2006 DE 102006062384
(43) Veröffentlichungstag der Anmeldung: 25.06.2008
(73) Patentinhaber: Aesculap AG, 78532 Tuttlingen (DE)
(72) Erfinder: Goldmann, Helmut, 78532 Tuttlingen (DE); Merckle, Christof, 68199 Mannheim (DE); Sievers, Hans-Hinrich, 24119 Kronshagen (DE)
(74) Vertreter: Patentanwälte Ruff, Wilhelm, Beier, Dauster & Partner

(56) Entgegenhaltungen:
- EP-A- 0 955 019
- WO-A-90/14804
- WO-A-2005/099624
- US-A1- 2004 215 333

## Beschreibung

Die vorliegende Erfindung betrifft eine schlauchförmige Gefäßprothese zum Ersatz der Aorta ascendens.

Der Ersatz von herznahen Bereichen der Aorta, insbesondere der Aortenwurzel und der Aorta ascendens, durch alloplastische Implantate gehört zu den häufigsten Operationen der Aortenchirurgie. Hierfür stehen in der Zwischenzeit eine Vielzahl von Gefäßprothesen zur Verfügung. Ist die Aortenwurzel betroffen, werden Gefäßprothesen gewöhnlich nach Entfernung von aneurysmatischen Teilen oder Dissektionen der Aorta und insbesondere nach Entfernung der Aortensinus (natürlichen Sinus) unter Erhalt der Aortenklappen und Teilen der Arterienwand implantiert.

Bei der ersten Generation von Gefäßprothesen handelte es sich um röhrenförmige Prothesen, wobei es sich bei den Röhren um gerade und homogene Röhren handelte. Diese Gefäßprothesen besitzen jedoch den Nachteil, dass ihnen zusätzliche Strukturen fehlen, welche insbesondere zum Ersatz von operativ entfernten Aortensinus geeignet sind. Demnach spiegeln sie die tatsächlichen anatomischen Gegebenheiten der Aorta ascendens nur unzureichend wieder.

Aus der US 6,544,285 B1 sowie der WO 01/52776 A1 sind röhrenförmige Gefäßprothesen für den Ersatz der Aorta ascendens bekannt, welche an ihrem unteren Ende (kaudalen Ende) künstliche Sinus zum Ersatz der natürlichen Aortensinus aufweisen. Die künstlichen Sinus sind an die Prothese angenäht. Durch die angenähten Sinus entstehen am kaudalen Ende der Gefäßprothese bogenförmige Ausbuchtungen. Zwar erlauben diese Gefäßprothesen grundsätzlich eine verbesserte Abbildung der tatsächlichen anatomischen Gegebenheiten der Aorta ascendens, doch gestaltet sich ihre Implantation in vielen Fällen als aufwändig.

In der EP 0 955 019 A2 werden röhrenförmige Gefäßprothesen beschrieben, welche im Bereich des Aortenbulbus eine generelle circumferentielle Ausbuchtung aufweisen. Diese Prothesenkonfiguration wird der tatsächlich vorliegenden Anatomie der Aorta ascendens ebenfalls nicht gerecht. Eine in dieser Hinsicht deutlich verbesserte röhrenförmige Gefäßprothese wird in der WO 2004/021925 A2 beschrieben, wonach die Röhre im herznahen Bereich, d.h. im Bereich der Aorta ascendens, mindestens zwei künstliche Sinus aufweist, die eine größere Ausdehnung nach außen besitzen als die Röhre.

In der US 2004/0215333 A1 wird eine Herzklappenprothese offenbart, welche zur Aufnahme in einen endovaskulären Stent vorgesehen ist. Die Herzklappenprothese weist zwei ringförmige Halterungen auf, welche über drei äquidistante Nähte miteinander verbunden sind.

Aufgabe der vorliegenden Erfindung ist es, eine Gefäßprothese zum Ersatz der Aorta ascendens bereitzustellen, welche aus dem Stand der Technik bekannte Nachteile vermeidet und insbesondere eine Konfiguration besitzt, welche einerseits die natürlichen anatomischen Gegebenheiten der Aorta ascendens in optimaler Weise widerspiegelt und andererseits bei der Implantation verbesserte Fixierungsmöglichkeiten der Prothese erlaubt.

Diese Aufgabe wird erfindungsgemäß durch eine schlauchförmige Gefäßprothese gemäß Anspruch 1 zum Ersatz der Aorta ascendens, insbesondere zum Ersatz der Aortenwurzel, gelöst, wobei die Gefäßprothese im Aortensinusbereich einen im wesentlichen geradlinig verlaufenden Wandabschnitt mit drei Umfangsabschnitten aufweist und zwischen den Umfangsabschnitten längsverlaufende Verstärkungen ausgebildet sind. Bevorzugt besteht der Wandabschnitt aus den drei Umfangsabschnitten und den längsverlaufenden Verstärkungen.

Durch die erfindungsgemäße Gefäßprothese können die sogenannten Kommissuren (Streben) der natürlichen Aortenwand an der Innenseite der Prothese im Bereich der Verstärkungen zwischen den Umfangsabschnitten fixiert werden. Dadurch ist eine einfache und sichere Fixierung der erfindungsgemäßen Gefäßprothese bei ihrer Implantation möglich.

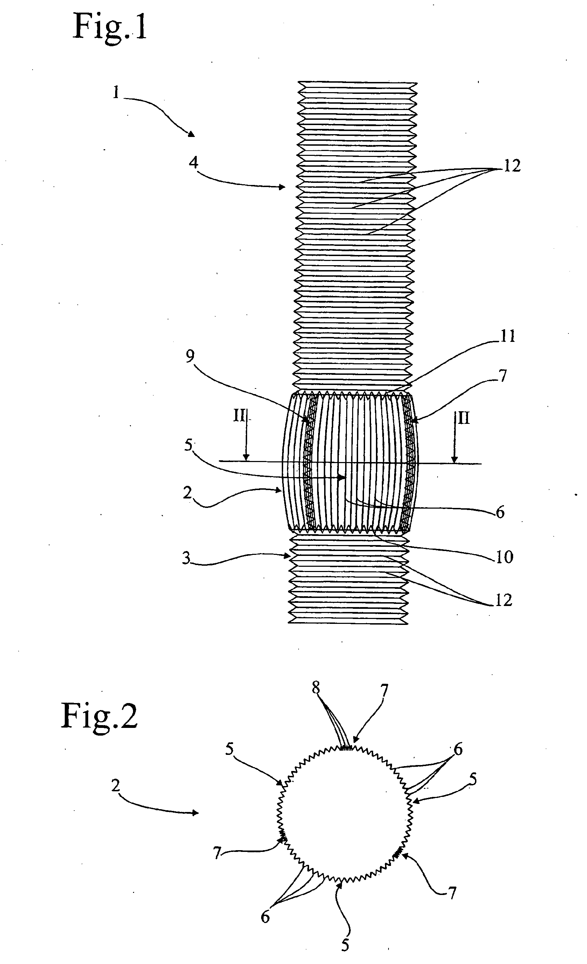

Ein im wesentlichen geradlinig verlaufender Wandabschnitt der erfindungsgemäßen Gefäßprothese bedeutet, dass die Gefäßprothese einen Verlauf aufweist, welcher idealer Weise dem natürlichen Verlauf der Aorta ascendens entspricht. Leichte Wölbungen des Wandabschnittes (vgl. Figur 1) sollen mitumfasst sein.

In einer bevorzugten Ausführungsform sind die Umfangsabschnitte in Querrichtung der Gefäßprothese erweiterbar. Auf diese Weise kann die natürliche Funktion von operativ entfernten natürlichen Sinus in optimaler Weise ersetzt werden. Die Umfangsabschnitte weisen vorzugsweise eine gegenüber der Gefäßprothese erhöhte Dehnbarkeit auf. Dadurch kann insbesondere eine Behinderung der Segelbewegung der Herzklappen vermieden werden. Weiterhin kann die erfindungsgemäße Gefäßprothese während der systolischen Herzbewegung ein größeres Blutvolumen aufnehmen.

Die erfindungsgemäße Gefäßprothese ist bevorzugt zumindest teilweise plissiert. Erfindungsgemäß kann es vorgesehen sein, das die Gefäßprothese vollständig plissiert ist. Es können insbesondere die Umfangsabschnitte und/oder die längsverlaufenden Verstärkungen der Gefäßprothese plissiert sein. Bevorzugt sind die Umfangsabschnitte und die Verstärkungen plissiert. Die zumindest teilweise Plissierung der Gefäßprothese liegt vorzugsweise in Form von Plissierfalten vor. Bei den Plissierfalten kann es sich insbesondere um längs- und/oder querverlaufende Plissierfalten handeln.

In einer bevorzugten Ausführungsform weisen die Umfangsabschnitte der Gefäßprothese längsverlaufende Plissierfalten auf. Dadurch sind die Umfangsabschnitte in besonders vorteilhafter Weise in Querrichtung der Gefäßprothese erweiterbar.

In einer anderen Ausführungsform sind die Umfangsabschnitte in Querrichtung der Gefäßprothese erweitert. Vorzugsweise sind die Umfangsabschnitte durch eine Aussackung der Prothesenwand im Aortensinusbereich erweitert.

Weiterhin können die Umfangsabschnitte aus Einzelstücken gebildet sein. Die Umfangsabschnitte können insbesondere in drei Aussparungen der Gefäßprothese direkt eingesetzt sein, wobei die Umfangsabschnitte zweckmäßigerweise bereits bei der Herstellung der Prothese dicht mit den sie umgebenden Prothesenwandteilen verbunden, insbesondere vernäht werden. Die Umfangsabschnitte können aus anderen Materialien gebildet sein als die übrigen Teile der Prothese.

In einer weitergehenden Ausführungsform sind die Umfangsabschnitte der Gefäßprothese einstückig miteinander ausgebildet. In einer weiteren Ausführungsform sind die Umfangsabschnitte und die längsverlaufenden Verstärkungen einstückig miteinander ausgebildet.

Die längsverlaufenden Verstärkungen der erfindungsgemäßen Gefäßprothese können einen im wesentlichen geradlinigen bis leicht gewölbten Verlauf aufweisen. Vorzugsweise weisen die längsverlaufenden Verstärkungen einen im wesentlichen geradlinigen Verlauf auf. Besonders bevorzugt entspricht der Verlauf der Verstärkungen in etwa dem Verlauf der Kommissuren in der natürlichen Aortenwand. Dies erleichtert die Fixierung der erfindungsgemäßen Gefäßprothese an die Kommissuren.

In einer bevorzugten Ausführungsform sind die längsverlaufenden Verstärkungen flächig, insbesondere steg- oder streifenförmig, in der Wandungsfläche der Gefäßprothese ausgebildet. Die längsverlaufenden Verstärkungen können insbesondere länger als der Prothesenwandabschnitt im Aortensinusbereich sein.

Bevorzugt ist der Prothesenwandabschnitt im Aortensinusbereich an seiner Außenseite mit den längsverlaufenden Verstärkungen versehen. Bei den längsverlaufenden Verstärkungen kann es sich insbesondere um vorgefertigte Verstärkungen handeln, welche auf der Außenseite der Prothese aufgebracht sind. Die längsverlaufenden Verstärkungen können insbesondere als Materialstreifen ausgebildet sein. Bevorzugt sind die längsverlaufenden Verstärkungen als sich mit dem Material der Umfangsabschnitte überlappende Materialstreifen ausgebildet. Die Verstärkungen sind zweckmäßigerweise durch geeignete Fixierungstechniken auf der Außenseite des Prothesenwandabschnittes aufgebracht. Als Fixierungstechniken kommen insbesondere Vernähen, Verkleben oder Verschweißen in Betracht.

Die längsverlaufenden Verstärkungen sind vorzugsweise aus polymeren Materialien gebildet. Bei den Materialien kann es sich um textile oder nicht textile Materialien handeln. Als polymere Materialien kommen insbesondere Polyester oder Polyurethane in Frage. Beispielsweise handelt es sich bei den Polyestern um Polyethylen- und/oder Polybutylenterephthalat. Bei den Polyurethanen handelt es sich vorzugsweise um aliphatische Polyurethane. Die Polyurethane können verzweigt oder unverzweigt sein. Vorzugsweise sind die Polyurethane unverzweigt.

In einer weiteren Ausführungsform besitzen die längsverlaufenden Verstärkungen jeweils eine Länge von 2 bis 8 cm, insbesondere von 2 bis 5 cm, beispielsweise von 2,5 bis 3,5 cm. Vorzugsweise besitzen die längsverlaufenden Verstärkungen jeweils eine Länge von 3 bis 4 cm. Die längsverlaufenden Verstärkungen besitzen jeweils bevorzugt eine Breite von 1 bis 10 mm, insbesondere von 2 bis 6 mm.

Vorzugsweise weisen die längsverlaufenden Verstärkungen längsverlaufende Plissierfalten auf. Die längsverlaufenden Verstärkungen weisen bevorzugt 2 bis 14, insbesondere 4 bis 10, vorzugsweise 6 bis 8, längsverlaufende Plissierfalten auf.

Die längsverlaufenden Verstärkungen weisen in Umfangsrichtung verdichtete längsverlaufende Plissierfalten auf. Bevorzugt sind die längsverlaufenden Verstärkungen aus verdichteten längsverlaufenden Plissierfalten gebildet. Beispielsweise können die Verstärkungen durch Zusammenraffen der längsverlaufenden Plissierfalten gebildet sein. Die längsverlaufenden Verstärkungen können zusätzlich eine Längsnaht aufweisen. Vorzugsweise sind die längsverlaufenden Verstärkungen im wesentlichen aus längsverlaufenden Plissierfalten und Nahtmaterial gebildet, das die Plissierfalten verdichtet und zusätzlich zur Verstärkung beiträgt.

In einer bevorzugten Ausführungsform sind die Umfangsabschnitte jeweils aus mindestens einem Materialstreifen gebildet. Die Materialstreifen können aus verschiedenen Materialien bestehen. Die Umfangsabschnitte können zudem verschiedene Formen aufweisen. Die Umfangsabschnitte sind vorzugsweise tropfen-, schild- oder wappenförmig ausgebildet. Besonders bevorzugt weisen die Umfangsabschnitte eine Form auf, welche im wesentlichen der Form von natürlichen Sinus entspricht.

In einer geeigneten Ausführungsform besitzt die Gefäßprothese eine poröse Wandung. Dadurch wird das Einwachsen von Gewebe und insbesondere von kleinen Blutgefäßen in optimaler Weise unterstützt.

In einer weiteren Ausführungsform ist die Gefäßprothese aus einem textilen Material gebildet. Erfindungsgemäß sind insbesondere die Umfangsabschnitte und/oder die längsverlaufenden Verstärkungen aus einem textilen Material gebildet. Bei dem textilen Material kann es sich insbesondere um Polymere, vorzugsweise um synthetische Polymere, handeln. Als Polymere kommen insbesondere Polyester und Polytetrafluorethylen in Frage. Als Polyester kommen insbesondere Polyethylen-und/oder Polybutylenterephthalat in Betracht. Die erfindungsgemäße Gefäßprothese ist bevorzugt gewirkt oder gewebt.

In einer weitergehenden Ausführungsform sind die längsverlaufenden Verstärkungen aus dem gleichen Material wie der Prothesenwandabschnitt im Aortensinusbereich, insbesondere wie die Umfangsabschnitte, gebildet. Bevorzugt weisen die längsverlaufenden Verstärkungen ihrerseits Verstärkungs- oder Versteifungselemente auf. Die Verstärkungs- bzw. Versteifungselemente sind vorteilhafterweise flächig ausgebildet. Bevorzugt sind die Verstärkungs- bzw. Versteifungselemente in die längsverlaufenden Verstärkungen der Gefäßprothese eingearbeitet. Besonders bevorzugt werden die Verstärkungs- bzw. Versteifungselemente vollständig von den längsverlaufenden Verstärkungen der Gefäßprothese umhüllt. Die Verstärkungs- bzw. Versteifungselemente sind insbesondere aus Metallen und/oder Kunststoffen gebildet. Vorzugsweise liegen die Verstärkungs- bzw. Versteifungselemente in Form von Drähten oder Streifen vor.

In einer weiteren Ausführungsform besitzt der Prothesenwandabschnitt im Aortensinusbereich eine Länge von 2 bis 5 cm, insbesondere von 2,5 bis 3,5 cm.

Erfindungsgemäß ist es insbesondere vorgesehen, dass die Gefäßprothese in Längsrichtung aus mindestens einem Wandabschnitt, insbesondere aus zwei Wandabschnitten, vorzugsweise aus drei Wandabschnitten, zusammengesetzt ist. Die einzelnen Wandabschnitte sind zweckmäßigerweise dicht miteinander verbunden. Vorzugsweise sind die einzelnen Wandabschnitte miteinander vernäht.

In einer bevorzugten Ausführungsform besteht die erfindungsgemäße Gefäßprothese aus mindestens einem Wandabschnitt, insbesondere aus zwei Wandabschnitten, vorzugsweise aus drei Wandabschnitten.

In einer weitergehenden Ausführungsform besitzt die Gefäßprothese an ihrem kaudalen Ende im Bereich der Aortenwurzel einen im wesentlichen geradlinigen Wandabschnitt, welcher sich an den Prothesenwandabschnitt im Aortensinusbereich anschließt. Der Prothesenwandabschnitt im Bereich der Aortenwurzel besitzt vorzugsweise eine Länge von 0,5 bis 3 cm, insbesondere von ca. 1 cm.

In einer weiteren bevorzugten Ausführungsform besitzt die Gefäßprothese an ihrem kranialen Ende im Aortenbogen nahen Bereich einen im wesentlichen geradlinigen Wandabschnitt, welcher sich an den Prothesenwandabschnitt im Aortensinusbereich anschließt. Der Prothesenwandabschnitt im Aortenbogen nahen Bereich besitzt vorzugsweise eine Länge von 2 bis 10 cm, insbesondere von ca. 5 cm. Der Prothesenwandabschnitt im Aortenbogen nahen Bereich kann Teil eines Prothesenstückes zum Ersatz des Aortenbogens und insbesondere zum Ersatz zumindest eines Teils der Aorta descendens sein. Das Prothesenstück kann insbesondere U-förmig ausgebildet sein.

Der Prothesenwandabschnitt im Bereich der Aortenbasis (Annulus) und/oder der Prothesenwandabschnitt im Aortenbogen nahen Bereich können plissiert oder unplissiert sein. Bevorzugt ist der Prothesenwandabschnitt im Bereich der Aortenwurzel und/oder der Prothesenwandabschnitt im Aortenbogen nahen Bereich plissiert. Die Plissierung liegt gewöhnlich in Form von Plissierfalten vor, wobei es sich bei den Plissierfalten vorzugsweise um querverlaufende (in Querrichtung der Gefäßprothese verlaufende) Plissierfalten handelt.

Weiterhin weist die erfindungsgemäße Gefäßprothese vorzugsweise einen im wesentlichen geradlinigen bis leicht gewölbten Verlauf auf. Die Gefäßprothese besitzt bevorzugt einen Innendurchmesser von 22 bis 40 mm, insbesondere von 26 bis 34 mm. Es ist ferner bevorzugt, dass die Gefäßprothese abdichtend imprägniert ist. Als Materialien zur Imprägnierung kommen insbesondere natürliche oder synthetische, insbesondere resorbierbare, Materialien in Betracht. Bei den Materialien handelt es sich vorzugsweise um resorbierbare Materialien. Für die Imprägnierung der Gefäßprothese kommen beispielsweise Polymere, insbesondere synthetische Polymere, in Frage. Beispielsweise kann die erfindungsgemäße Gefäßprothese mit Gelatine, insbesondere mit vernetzter Gelatine, abdichtend imprägniert sein. Weiterhin kann die Gefäßprothese mit einem Copolymer, insbesondere mit einem Ter- oder Tetrapolymer, abdichtend imprägniert sein. Bei dem Terpolymer kann es sich beispielsweise um ein Terpolymer auf der Basis von Glykolid, Trimethylencarbonat und ε-Caprolacton handeln.

In einer weiteren Ausführungsform liegt die Gefäßprothese in steriler Form vor. Erfindungsgemäß ist es insbesondere vorgesehen, dass die Gefäßprothese konfektioniert ist.

Weitere Einzelheiten und Merkmale der Erfindung ergeben sich aus den nachfolgenden Figurenbeschreibungen sowie der nachfolgenden Beschreibung von bevorzugten Ausführungsformen in Form von Beispielen in Verbindung mit den Unteransprüchen. Hierbei können die jeweiligen Merkmale für sich alleine oder zu mehreren in Kombination miteinander verwirklicht sein.

In den Zeichnungen zeigen:
- Figur 1:: eine Seitenansicht einer Ausführungsform der Erfindung und
- Figur 2:: einen Schnitt entlang der Linie II-II nach Figur 1.

### Figurenbeschreibung

In der schematischen Seitenansicht der Figur 1 bzw. in der schematischen Schnittansicht der Figur 2 (Schnitt entlang der Linie II-II nach Figur 1) ist eine erfindungsgemäße Gefäßprothese 1 gezeigt, welche aus insgesamt drei Wandungsabschnitten 2; 3; 4 zusammengesetzt ist. Die Gefäßprothese 1 ist aus einem gewebten multifilen Garn hergestellt. Der Wandabschnitt 2 liegt nach Implantation der Gefäßprothese 1 im Aortensinusbereich des Herzens. Der Prothesenwandabschnitt 2 weist einen im wesentlichen geradlinigen bis leicht gewölbten Verlauf auf mit drei in Querrichtung der Gefäßprothese 1 erweiterbaren Umfangsabschnitten 5, die, wie aus Figur 2 ersichtlich, um 120° versetzt zueinander angeordnet sind. Die erweiterbaren Umfangsabschnitte 5 weisen ihrerseits längsverlaufende Plissierfalten 6 auf. Zwischen den erweiterbaren Umfangsabschnitten 5 sind drei längsverlaufende Verstärkungen 7 ausgebildet. Die längsverlaufenden Verstärkungen 7 bestehen jeweils aus sechs zusammengerafften Plissierfalten 8, welche senkrecht mit einem geeigneten Nahtmaterial 9 abgenäht sind. Bei dem Nahtmaterial 9 handelt es sich beispielsweise um das unter der Bezeichnung Premicron^{®} kommerziell erhältliche Nahtmaterial. Der Prothesenwandabschnitt 2 weist eine Länge von 30 mm auf. An seinem unteren Ende schließt sich ein weiterer Wandabschnitt 3 der Gefäßprothese 1 an. Die Prothesenwandabschnitte 2 und 3 sind über eine in Querrichtung der Gefäßprothese 1 verlaufende Naht 10 miteinander vernäht. Der Prothesenwandabschnitt 3 liegt nach Implantation der Gefäßprothese 1 im herznahen Bereich, d.h. im Bereich der Aortenbasis (Annulus). Der Prothesenwandabschnitt 3 weist ebenfalls einen im wesentlichen geradlinigen Verlauf auf und besitzt eine Länge von ca. 10 mm. Durch den geradlinigen Verlauf des Prothesenwandabschnittes 3 kann die Gefäßprothese 1 während der Operation in einfacher Weise an der Aortenwurzel des Herzens angenäht werden. Am oberen Ende des Prothesenwandabschnittes 2 schließt sich ein weiterer Prothesenwandabschnitt 4 an. Dieser Wandabschnitt liegt nach Implantation der Gefäßprothese 1 im Bereich der Aorta ascendens. Der Prothesenwandabschnitt 4 besitzt ebenso einen im wesentlichen geradlinigen Verlauf. Die Länge des Prothesenwandabschnittes 4 beträgt mindestens ca. 20 mm. Der Prothesenwandabschnitt 4 kann auch den Aortenbogen mit umfassen.

Die Prothesenwandabschnitte 2 und 4 sind über eine in Querrichtung der Gefäßprothese 1 verlaufende Naht 11 miteinander vernäht. Die Prothesenwandabschnitte 3 und 4 weisen jeweils in Querrichtung der Gefäßprothese 1 verlaufende Plissierfalten 12 auf. Dadurch wird eine optimale Dehnbarkeit der Gefäßprothese 1 in ihrer Längsrichtung ermöglicht, wohingegen die in Längsrichtung verlaufenden Plissierfalten 6 und 8 des Prothesenwandabschnittes 2 eine optimale Dehnbarkeit in Querrichtung der Gefäßprothese 1 während der systolischen Herzbewegung gestatten.

Die in den Figuren 1 und 2 schematisch wiedergegebene Gefäßprothese 1 eignet sich in besonderer Weise zum Ersatz der Aortenwurzel und der Aorta ascendens, insbesondere einschließlich von operativ entfernten natürlichen Sinus. Durch die Verstärkungen 7 des Prothesenwandabschnittes 2 können die Kommissuren der natürlichen Aortenwand an der Innenseite der Gefäßprothese 1 im Bereich der Verstärkungen 7 fixiert werden. Dadurch ist eine im Vergleich zu den aus dem Stand der Technik bekannten Gefäßprothesen verbesserte Verankerung bzw. Fixierung der Gefäßprothese 1 bei ihrer Implantation möglich. Die Verankerungsmöglichkeiten der Gefäßprothese 1 können zusätzlich durch den im wesentlichen geradlinig verlaufenden Wandabschnitt 3 verbessert werden.

### Herstellungsbeispiele für erfindungsgemäße Gefäßprothese

### Beispiel 1: Herstellung einer Sinus-Wurzel-Prothese in mehreren Nähschritten

Eine schlauchförmige und plissierte gewebte Gefäßprothese mit einem Durchmesser von ca. 3 cm wird in ein 3 cm und in ein 1 cm langes Lumenstück geschnitten. Darüber hinaus wird eine 12 cm lange und 3 cm breite Bahn quer zu ihrer Plissierung mit einem Laser herausgeschnitten. Diese Bahn wird anschließend zu einem Lumenstück mit einem Durchmesser von ca. 4 cm zusammengenäht. Anschließend werden 3 Mal je sechs Plissierfalten der Bahn zusammengerafft und mit dem unter der Bezeichnung Premicron^{®} kommerziell erhältlichen Nähfaden senkrecht abgenäht, wobei die schon vorhandene Naht vorzugsweise miteinbezogen wird. In die Nähte können zusätzliche Verstärkungselemente wie Drahtstücke aus Kunststoff auf der Wandungsaußenseite miteingenäht werden. Durch die Zusammenraffung ergibt sich ein Lumenstück mit einem Durchmesser von ca. 3 cm. Dieses wird zwischen die beiden anderen Lumenstücke eingenäht. Schließlich wird die Prothese mit Gelatine beschichtet, wobei die Gelatine zusätzlich vernetzt wird. Die so hergestellte Gefäßprothese wird anschließend einer γ-Sterilisierung unterworfen.

## Patentansprüche

1. Schlauchförmige Gefäßprothese (1) zum Ersatz der Aorta ascendens, mit einem Aortensinusbereich, **dadurch gekennzeichnet, dass** die Gefäßprothese (1) im Aortensinusbereich einen im wesentlichen geradlinig verlaufenden Wandabschnitt (2) mit drei Umfangsabschnitten (5) aufweist und zwischen den Umfangsabschnitten (5) längsverlaufende Verstärkungen (7) ausgebildet sind und die längsverlaufenden Verstärkungen (7) in Umfangsrichtung verdichtete längsverlaufende Plissierfalten aufweisen.

2. Gefäßprothese (1) nach Anspruch 1, **dadurch gekennzeichnet, dass** die Umfangsabschnitte (5) in Querrichtung der Gefäßprothese (1) erweiterbar sind.

3. Gefäßprothese (1) nach Anspruch 1 oder 2, **dadurch gekennzeichnet, dass** die Umfangsabschnitte (5) eine gegenüber der Gefäßprothese (1) erhöhte Dehnbarkeit besitzen.

4. Gefäßprothese (1) nach einem der vorhergehenden Ansprüche, **dadurch gekennzeichnet, dass** die Umfangsabschnitte (5) längsverlaufende Plissierfalten (6) aufweisen.

5. Gefäßprothese (1) nach einem der vorhergehenden Ansprüche, **dadurch gekennzeichnet, dass** die Umfangsabschnitte (5) einstückig miteinander ausgebildet sind.

6. Gefäßprothese (1) nach einem der vorhergehenden Ansprüche, **dadurch gekennzeichnet, dass** die Umfangsabschnitte (5) und die längsverlaufenden Verstärkungen (7) einstückig miteinander ausgebildet sind.

7. Gefäßprothese (1) nach einem der vorhergehenden Ansprüche, **dadurch gekennzeichnet, dass** die längsverlaufenden Verstärkungen (7) einen im Wesentlichen geradlinigen Verlauf aufweisen.

8. Gefäßprothese (1) nach einem der vorhergehenden Ansprüche, **dadurch gekennzeichnet, dass** die längsverlaufenden Verstärkungen (7) flächig, insbesondere stegförmig, in der Wandungsfläche der Gefäßprothese (1) ausgebildet sind.

9. Gefäßprothese (1) nach einem der vorhergehenden Ansprüche, **dadurch gekennzeichnet, dass** der Prothesenwandabschnitt (2) im Aortensinusbereich an seiner Außenseite mit den längsverlaufenden Verstärkungen (7) versehen ist.

10. Gefäßprothese (1) nach einem der vorhergehenden Ansprüche, **dadurch gekennzeichnet, dass** die längsverlaufenden Verstärkungen (7) jeweils eine Länge von 2 bis 8 cm, insbesondere von 2 bis 5 cm, vorzugsweise von 3 bis 4 cm, besitzen.

11. Gefäßprothese (1) nach einem der vorhergehenden Ansprüche, **dadurch gekennzeichnet, dass** die längsverlaufenden Verstärkungen (7) jeweils eine Breite von 1 bis 10 mm, insbesondere von 2 bis 6 mm, besitzen.

12. Gefäßprothese (1) nach einem der vorhergehenden Ansprüche, **dadurch gekennzeichnet, dass** die längsverlaufenden Verstärkungen (7) eine Längsnaht aufweisen.

13. Gefäßprothese (1) nach einem der vorhergehenden Ansprüche, **dadurch gekennzeichnet, dass** die längsverlaufenden Verstärkungen (7) im wesentlichen aus längsverlaufenden Plissierfalten (8) und Nahtmaterial (9) gebildet sind.

14. Gefäßprothese (1) nach einem der vorhergehenden Ansprüche, **dadurch gekennzeichnet, dass** die Umfangsabschnitte (5) jeweils aus mindestens einem Materialstreifen gebildet sind.

15. Gefäßprothese (1) nach einem der vorhergehenden Ansprüche, **dadurch gekennzeichnet, dass** die Gefäßprothese (1), insbesondere die Umfangsabschnitte (5) und/oder die längsverlaufenden Verstärkungen (7), aus einem textilen Material gebildet ist.

16. Gefäßprothese (1) nach einem der vorhergehenden Ansprüche, **dadurch gekennzeichnet, dass** die längsverlaufenden Verstärkungen (7) aus dem gleichen Material wie der Prothesenwandabschnitt (2) im Aortensinusbereich, insbesondere wie die Umfangsabschnitte (5), gebildet sind.

17. Gefäßprothese (1) nach einem der vorhergehenden Ansprüche, **dadurch gekennzeichnet, dass** die längsverlaufenden Verstärkungen (7) ihrerseits Verstärkungs- oder Versteifungselemente aufweisen.

18. Gefäßprothese (1) nach einem der vorhergehenden Ansprüche, **dadurch gekennzeichnet, dass** der Prothesenwandabschnitt (2) im Aortensinusbereich eine Länge von 2 bis 5 cm, insbesondere eine Länge von 2,5 bis 3,5 cm, besitzt.

19. Gefäßprothese (1) nach einem der vorhergehenden Ansprüche, **dadurch gekennzeichnet, dass** die Gefäßprothese (1) aus mindestens einem Wandabschnitt, insbesondere aus zwei, vorzugsweise aus drei, Wandabschnitten, zusammengesetzt ist.

20. Gefäßprothese (1) nach einem der vorhergehenden Ansprüche, **dadurch gekennzeichnet, dass** die Gefäßprothese (1) an ihrem unteren Ende im Bereich der Aortenbasis einen im wesentlichen geradlinigen Wandabschnitt (3) besitzt, welcher sich an den Prothesenwandabschnitt (2) im Aortensinusbereich anschließt.

21. Gefäßprothese (1) nach Anspruch 23, **dadurch gekennzeichnet, dass** der Prothesenwandabschnitt (3) im Bereich der Aortenwurzel eine Länge von 0,5 bis 3 cm, insbesondere von ca. 1 cm, besitzt.

22. Gefäßprothese (1) nach einem der vorhergehenden Ansprüche, **dadurch gekennzeichnet, dass** die Gefäßprothese (1) an ihrem kranialen Ende im Aortenbogen nahen Bereich einen im wesentlichen geradlinigen Wandabschnitt (4) besitzt, welcher sich an den Prothesenwandabschnitt (2) im Aortensinusbereich anschließt.

23. Gefäßprothese (1) nach Anspruch 25, **dadurch gekennzeichnet, dass** der Prothesenwandabschnitt (4) im Aortenbogen nahen Bereich eine Länge von 2 bis 10 cm, insbesondere von ca. 2 bis 4 cm, besitzt.

24. Gefäßprothese (1) nach einem der vorhergehenden Ansprüche, **dadurch gekennzeichnet, dass** die Gefäßprothese (1) einen Innendurchmesser von 22 bis 40 mm, insbesondere von 26 bis 34 mm, besitzt.

25. Gefäßprothese (1) nach einem der vorhergehenden Ansprüche, **dadurch gekennzeichnet, dass** die Gefäßprothese (1) abdichtend imprägniert ist.

## Claims

1. Vessel prosthesis (1) of tubular shape for the replacement of the ascending aorta, with an aortic sinus area, **characterized in that** the vessel prosthesis (1) has, in the aortic sinus area, a substantially rectilinear wall section (2) with three circumferential sections (5), and longitudinal reinforcements (7) are formed between the circumferential sections (5), and the longitudinal reinforcements (7) have longitudinal pleat folds compacted in the circumferential direction.

2. Vessel prosthesis (1) according to Claim 1, **characterized in that** the circumferential sections (5) can widen in the transverse direction of the vessel prosthesis (1).

3. Vessel prosthesis (1) according to Claim 1 or 2, **characterized in that** the circumferential sections (5) have increased expansibility in relation to the vessel prosthesis (1).

4. Vessel prosthesis (1) according to one of the preceding claims, **characterized in that** the circumferential sections (5) have longitudinal pleat folds (6).

5. Vessel prosthesis (1) according to one of the preceding claims, **characterized in that** the circumferential sections (5) are formed integrally with one another.

6. Vessel prosthesis (1) according to one of the preceding claims, **characterized in that** the circumferential sections (5) and the longitudinal reinforcements (7) are formed integrally with one another.

7. Vessel prosthesis (1) according to one of the preceding claims, **characterized in that** the longitudinal reinforcements (7) have a substantially rectilinear course.

8. Vessel prosthesis (1) according to one of the preceding claims, **characterized in that** the longitudinal reinforcements (7) have a planar, in particular web-shaped form in the wall surface of the vessel prosthesis (1).

9. Vessel prosthesis (1) according to one of the preceding claims, **characterized in that** the prosthesis wall section (2) in the aortic sinus area is provided on the outside thereof with the longitudinal reinforcements (7).

10. Vessel prosthesis (1) according to one of the preceding claims, **characterized in that** the longitudinal reinforcements (7) each have a length of 2 to 8 cm, in particular of 2 to 5 cm, preferably of 3 to 4 cm.

11. Vessel prosthesis (1) according to one of the preceding claims, **characterized in that** the longitudinal reinforcements (7) each have a width of 1 to 10 mm, in particular of 2 to 6 mm.

12. Vessel prosthesis (1) according to one of the preceding claims, **characterized in that** the longitudinal reinforcements (7) have a longitudinal seam.

13. Vessel prosthesis (1) according to one of the preceding claims, **characterized in that** the longitudinal reinforcements (7) are formed substantially of longitudinally extending pleat folds (8) and seam material (9).

14. Vessel prosthesis (1) according to one of the preceding claims, **characterized in that** the circumferential sections (5) are each formed of at least one material strip.

15. Vessel prosthesis (1) according to one of the preceding claims, **characterized in that** the vessel prosthesis (1), in particular the circumferential sections (5) and/or the longitudinal reinforcements (7), is/are made of a textile material.

16. Vessel prosthesis (1) according to one of the preceding claims, **characterized in that** the longitudinal reinforcements (7) are made of the same material as the prosthesis wall section (2) in the aortic sinus area, in particular of the same material as the circumferential sections (5).

17. Vessel prosthesis (1) according to one of the preceding claims, **characterized in that** the longitudinal reinforcements (7) in turn have reinforcing or stiffening elements.

18. Vessel prosthesis (1) according to one of the preceding claims, **characterized in that** the prosthesis wall section (2) in the aortic sinus area has a length of 2 to 5 cm, in particular a length of 2.5 to 3.5 cm.

19. Vessel prosthesis (1) according to one of the preceding claims, **characterized in that** the vessel prosthesis (1) is composed of at least one wall section, in particular of two and preferably of three wall sections.

20. Vessel prosthesis (1) according to one of the preceding claims, **characterized in that** the vessel prosthesis (1) has, at its lower end in the area of the aortic annulus, a substantially rectilinear wall section (3), which adjoins the prosthesis wall section (2) in the aortic sinus area.

21. Vessel prosthesis (1) according to Claim 20, **characterized in that** the prosthesis wall section (3), in the area of the aortic root, has a length of 0.5 to 3 cm, in particular of ca 1 cm.

22. Vessel prosthesis (1) according to one of the preceding claims, **characterized in that** the vessel prosthesis (1) has, at its cranial end near the aortic arch, a substantially rectilinear wall section (4), which adjoins the prosthesis wall section (2) in the aortic sinus area.

23. Vessel prosthesis (1) according to Claim 22, **characterized in that** the prosthesis wall section (4) in the area near the aortic arch has a length of 2 to 10 cm, in particular of ca 2 to 4 cm.

24. Vessel prosthesis (1) according to one of the preceding claims, **characterized in that** the vessel prosthesis (1) has an internal diameter of 22 to 40 mm, in particular of 26 to 34 mm.

25. Vessel prosthesis (1) according to one of the preceding claims, **characterized in that** the vessel prosthesis (1) is sealingly impregnated.

## Revendications

1. Prothèse vasculaire (1) en forme de tuyau souple destinée à remplacer l'aorte ascendante comprenant une zone de sinus aortique, **caractérisée en ce que** la prothèse vasculaire (1) présente dans la zone de sinus aortique une section de paroi (2) au tracé essentiellement linéaire comprenant trois sections circonférentielles (5) et des renforcements s'étendant longitudinalement (7) sont formés entre les sections circonférentielles (5) et les renforcements s'étendant longitudinalement (7) présentent des plis de plissage s'étendant longitudinalement et compactés dans la direction circonférentielle.

2. Prothèse vasculaire (1) selon la revendication 1, **caractérisée en ce que** les sections circonférentielles (5) sont extensibles dans la direction transversale de la prothèse vasculaire (1).

3. Prothèse vasculaire (1) selon la revendication 1 ou 2, **caractérisée en ce que** les sections circonférentielles (5) possèdent une extensibilité élevée par rapport à la prothèse vasculaire (1).

4. Prothèse vasculaire (1) selon l'une des revendications précédentes, **caractérisée en ce que** les sections circonférentielles (5) présentent des plis de plissage s'étendant longitudinalement (6).

5. Prothèse vasculaire (1) selon l'une des revendications précédentes, **caractérisée en ce que** les sections circonférentielles (5) sont formées d'un seul tenant les unes avec les autres.

6. Prothèse vasculaire (1) selon l'une des revendications précédentes, **caractérisée en ce que** les sections circonférentielles (5) et les renforcements s'étendant longitudinalement (7) sont formés d'un seul tenant les uns avec les autres.

7. Prothèse vasculaire (1) selon l'une des revendications précédentes, **caractérisée en ce que** les renforcements s'étendant longitudinalement (7) présentent un tracé essentiellement linéaire.

8. Prothèse vasculaire (1) selon l'une des revendications précédentes, **caractérisée en ce que** les renforcements s'étendant longitudinalement (7) sont réalisés de manière plane, en particulier en forme de nervures, dans la surface de paroi de la prothèse vasculaire (1).

9. Prothèse vasculaire (1) selon l'une des revendications précédentes, **caractérisée en ce que** la section de paroi (2) de la prothèse dans la zone de sinus aortique, est munie des renforcements s'étendant longitudinalement (7) sur son côté extérieur.

10. Prothèse vasculaire (1) selon l'une des revendications précédentes, **caractérisée en ce que** les renforcements s'étendant longitudinalement (7) possèdent respectivement une longueur de 2 à 8 cm, en particulier de 2 à 5 cm, de préférence de 3 à 4 cm.

11. Prothèse vasculaire (1) selon l'une des revendications précédentes, **caractérisée en ce que** les renforcements s'étendant longitudinalement (7) possèdent respectivement une largeur de 1 à 10 mm, en particulier de 2 à 6 mm.

12. Prothèse vasculaire (1) selon l'une des revendications précédentes, **caractérisée en ce que** les renforcements s'étendant longitudinalement (7) présentent un joint longitudinal.

13. Prothèse vasculaire (1) selon l'une des revendications précédentes, **caractérisée en ce que** les renforcements s'étendant longitudinalement (7) sont formés essentiellement de plis de plissage s'étendant longitudinalement (8) et de matériau de joint (9).

14. Prothèse vasculaire (1) selon l'une des revendications précédentes, **caractérisée en ce que** les sections circonférentielles (5) sont formées respectivement d'au moins une bande de matériau.

15. Prothèse vasculaire (1) selon l'une des revendications précédentes, **caractérisée en ce que** la prothèse vasculaire (1), en particulier les sections circonférentielles (5) et/ou les renforcements s'étendant longitudinalement (7) sont formé(e)s d'un matériau textile.

16. Prothèse vasculaire (1) selon l'une des revendications précédentes, **caractérisée en ce que** les renforcements s'étendant longitudinalement (7) sont fabriqués dans le même matériau que la section de paroi (2) de la prothèse dans la zone de sinus aortique, en particulier dans le même matériau que les sections circonférentielles (5).

17. Prothèse vasculaire (1) selon l'une des revendications précédentes, **caractérisée en ce que** les renforcements s'étendant longitudinalement (7) présentent pour leur part des éléments de renforcement ou de raidissement.

18. Prothèse vasculaire (1) selon l'une des revendications précédentes, **caractérisée en ce que** la section de paroi (2) de la prothèse dans la zone de sinus aortique possède une longueur de 2 à 5 cm, en particulier une longueur de 2,5 à 3,5 cm.

19. Prothèse vasculaire (1) selon l'une des revendications précédentes, **caractérisée en ce que** la prothèse vasculaire (1) est composée d'au moins une section de paroi, en particulier de deux, de préférence de trois sections de paroi.

20. Prothèse vasculaire (1) selon l'une des revendications précédentes, **caractérisée en ce que** la prothèse vasculaire (1) possède sur son extrémité inférieure au niveau de la base de l'aorte, une section de paroi (3) essentiellement linéaire qui se raccorde à la section de paroi (2) de la prothèse dans la zone de sinus aortique.

21. Prothèse vasculaire (1) selon la revendication 20, **caractérisée en ce que** la section de paroi (3) de la prothèse au niveau de la racine aortique possède une longueur de 0,5 à 3 cm, en particulier d'environ 1 cm.

22. Prothèse vasculaire (1) selon l'une des revendications précédentes, **caractérisée en ce que** la prothèse vasculaire (1) possède sur son extrémité crânienne dans la zone proche de l'arc aortique, une section de paroi (4) essentiellement linéaire qui se raccorde à la section de paroi (2) de la prothèse dans la zone de sinus aortique.

23. Prothèse vasculaire (1) selon la revendication 22, **caractérisée en ce que** la section de paroi (4) de la prothèse dans la zone proche de l'arc aortique, possède une longueur de 2 à 10 cm, en particulier d'environ 2 à 4 cm.

24. Prothèse vasculaire (1) selon l'une des revendications précédentes, **caractérisée en ce que** la prothèse vasculaire (1) possède un diamètre intérieur de 22 à 40 mm, en particulier de 26 à 34 mm.

25. Prothèse vasculaire (1) selon l'une des revendications précédentes, **caractérisée en ce que** la prothèse vasculaire (1) est imprégnée pour être étanche.
